# EUROPEAN PATENT APPLICATION

(11) **EP 2 457 898 A1**
(43) Date of publication of application: **30.05.2012**
(21) Application number: 10802209.6
(22) Date of filing: 08.07.2010
(51) Int. Cl.: C07D 209/70, C07D 487/04, C07D 487/14, C07D 487/22, H01L 51/42, C07C 13/64, C07C 69/618

(54) **FULLERENE DERIVATIVE**

(30) Priority: 22.07.2009 JP 2009170902; 04.09.2009 JP 2009204497; 16.11.2009 JP 2009260724
(71) Applicant: Sumitomo Chemical Company Limited, Tokyo 104-8260 (JP)
(72) Inventor: UETANI, Yasunori, Tsukuba-shi Ibaraki 305-0045 (JP); FUJIWARA, Jun, Ashiya-shi Hyogo 659-0055 (JP)
(74) Representative: Vossius & Partner
(86) International application number: PCT/JP2010/061931
(87) International publication number: WO 2011/010589

(57) **Abstract**

An organic photoelectronic conversion element having a sufficiently high open-circuit voltage can be produced by using a fullerene derivative comprising a structure represented by formula (1) and one or more structures selected from the group consisting of a structure represented by formula (2-1) and a structure represented by formula (2-2). 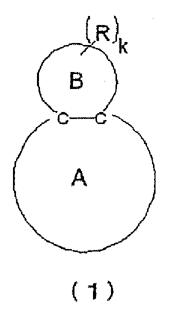 [wherein, ring A represents a fullerene skeleton, ring B represents a heterocyclic ring having a carbon number of 3 to 6, R represents a monovalent group, and k represents an integer of 1 to 8; and when there is a plurality of Rs, the Rs may be the same or different.] 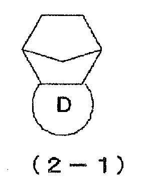 [wherein, ring D represents an aromatic ring.] 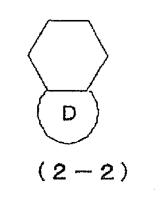 [wherein, ring D represents an aromatic ring.]

## Description

### Technical Field

The present invention relates to a fullerene derivative and an organic photoelectric conversion element using the same.

### Background Art

Organic semiconductor materials having charge (electron and hole) transport properties have been studied for application to organic photoelectric conversion element such as organic solar cells and optical sensors, and for example, fullerene derivatives, which are organic semiconductor materials, have been studied for application to organic solar cells. Example of fullerene derivative is [6.6]-phenyl C61-butyric acid methyl ester ([60]-PCBM) (see Advanced Functional Materials Vol.13 (2003) p.85).

### Summery of the Invention

However, there is a problem that an organic photoelectric conversion element comprising [60]-PCBM does not necessarily have a sufficient open circuit voltage (Voc).

The present invention provides a fullerene derivative capable of preparing an organic photoelectric conversion element having a sufficiently high open circuit voltage (Voc).

More specifically, the present invention provides a fullerene derivative having a structure represented by formula (1) and one or more structures selected from the group consisting of a structure represented by formula (2-1) and a structure represented by formula (2-2). 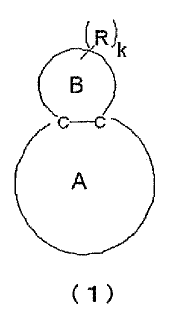 [wherein, ring A represents a fullerene skeleton, ring B represents a heterocyclic ring having a carbon number of 3 to 6, R represents a monovalent group, and k represents an integer of 1 to 8; and when there is a plurality of Rs, the Rs may be the same or different.] 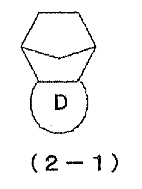 [wherein, ring D represents an aromatic ring.] 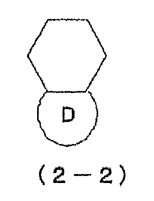 [wherein, ring D represents an aromatic ring.]

In addition, the present invention provides fullerene derivatives represented by formula (3-1), formula (3-2), formula (3-3), and formula (3-4) described below. 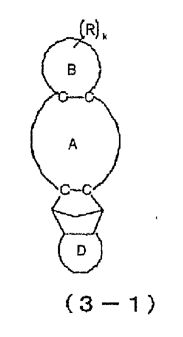 [wherein, ring A, ring B, ring D, R, and k represent as defined above.] 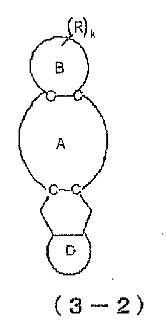 [wherein, ring A, ring B, ring D, R, and k represent as defined above.] 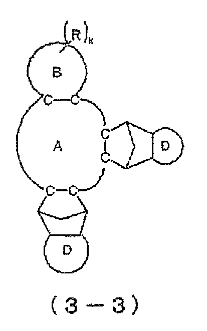 [wherein, ring A, ring B, ring D, R, and k represent as defined above, and a plurality of rings D may be the same or different.] 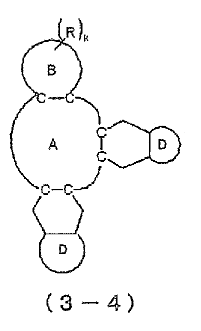 [wherein, ring A, ring B, ring D, R, and k represent as defined above, and a plurality of rings D may be the same or different.]

### Modes for Carrying Out the Invention

Hereinafter, the present invention is described in detail.

The fullerene derivative of the present invention has a structure represented by formula (1) and one or more structures selected from the group consisting of a structure represented by the formula (2-1) and a structure represented by formula (2-2). R in the formula (1) represents a monovalent group. The monovalent group is preferably an alkyl group, an alkoxy group, an aryl group, a halogen atom, a heterocyclic group, and a group having an ester structure. In addition, when there is a plurality of Rs, the Rs may be the same or different.

The alkyl group represented by R generally has 1 to 20 carbon atoms, may be linear or branched, and may be cyclic (cycloalkyl). Specific examples of the alkyl group include a methyl group, an ethyl group, a propyl group, an isopropyl group, a butyl group, an isobutyl group, a tert-butyl group, a sec-butyl group, a 3-methylbutyl group, a pentyl group, a hexyl group, a 2-ethylhexyl group, a heptyl group, an octyl group, a nonyl group, a decyl group, and a lauryl group. The monovalent group may be a group in which a hydrogen atom(s) in the above alkyl group is substituted with a halogen atom(s) and specifically includes a monohalomethyl group, a dihalomethyl group, a trihalomethyl group, and a pentahaloethyl group. Among halogen atoms, a fluorine atom is preferable. Specific examples of the alkyl group in which a hydrogen atom(s) is substituted with a fluorine atom(s) include a trifluoromethyl group, a pentafluoroethyl group, a perfluorobutyl group, a perfluorohexyl group, and a perfluorooctyl group. In addition, the monovalent group may be a group in which a hydrogen atom(s) in the above alkyl group is substituted with an alkoxy group(s), aryl group(s), heterocyclic group(s), or group(s) having an ester structure detailed below.

The alkoxy group represented by R generally has 1 to 20 carbon atoms, may be linear or branched, and may be a cycloalkyloxy group. Specific examples of the alkoxy group include a methoxy group, an ethoxy group, a propoxy group, an isopropoxy group, a butoxy group, an isobutoxy group, a sec-butoxy group, a tert-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, and a lauryloxy group. The monovalent group may be a group in which a hydrogen atom(s) in the above alkoxy group is substituted with a halogen atom(s). Among halogen atoms, a fluorine atom is preferable. Specific examples of the alkoxy group in which a hydrogen atom(s) is substituted with a fluorine atom(s) include a trifluoromethoxyl group, a pentafluoroethoxy group, a perfluorobutoxy group, a perfluorohexyloxy group, and a perfluorooctyloxy group. In addition, the monovalent group may be a group in which a hydrogen atom(s) in the above al koxy group is further substituted with an alkoxy group(s), aryl group(s), heterocyclic group(s), or group(s) having an ester structure detailed below.

The aryl group represented by R is a hydrocarbon group that is an aromatic group, has generally 6 to 60 carbon atoms and preferably 6 to 20 carbon atoms, and may have a substituent (s) such as the above alkyl group, the above alkoxy group or halogen atom, or the heterocyclic group or group having an ester structure detailed below. The substituent of the aryl group includes a linear, branched, or cyclic alkyl group having 1 to 20 carbon atoms, and an alkoxy group comprising in its structure a linear, branched, or cyclic alkyl group having 1 to 20 carbon atoms. Specific examples of the aryl group include a phenyl group, a C₁ to C₁₂ alkoxyphenyl group (C₁ to C₁₂ show that alkoxy has 1 to 12 carbon atoms.), a C₁ to C₁₂ alkylphenyl group (C₁ to C₁₂ show that alkyl has 1 to 12 carbon atoms.), a 1-naphthyl group, and a 2-naphthyl group, and a C₁ to C₁₂ alkoxyphenyl group and a C₁ to C₁₂ alkylphenyl group are more preferred. The substituent halogen atom is preferably a fluorine atom.

The halogen atom represented by R includes each of atoms of a fluorine atom, a chlorine atom, a bromine atom, and an iodine atom.

Examples of the heterocyclic group represented by R include a thienyl group, a pyridyl group, a furyl group, a quinolyl group, an isoquinolyl group, a pyrrolyl group, and a piperidyl group. The monovalent aromatic heterocyclic group is preferable. The heterocyclic group may be substituted with the above alkoxy group, the above aryl group or halogen atom, or the group having an ester structure detailed below.

Examples of the group having an ester structure include a group in which one hydrogen atom is removed from methyl butyrate, a group in which one hydrogen atom is removed from butyl butyrate, a group in which one hydrogen atom is removed from isopropyl butyrate, and a group in which one hydrogen atom is removed from 3-ethyl thienyl butyrate.

In addition, one embodiment of the group having an ester structure is a group represented by formula (6). 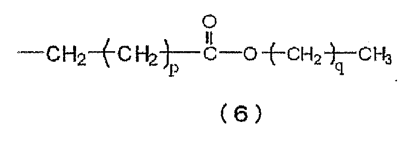 [wherein, p represents an integer of 0 to 10, and q is an integer of 1 to 10.]

In formula (1), ring A representing a fullerene skeleton is preferably C₆₀ fullerene skeleton or C₇₀ fullerene skeleton, from the viewpoint of the raw material availability.

Specific examples of ring B of formula (1) include the following rings.

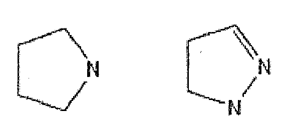

Ring B is preferably the following ring, from the viewpoint of ease of synthesis.

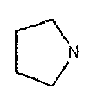

The structure represented by formula (1) is preferably a structure represented by formula (12). 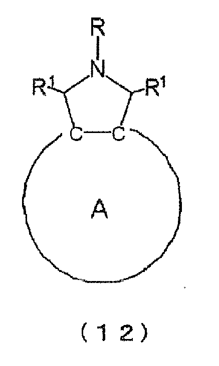 [wherein, ring A and R represent as defined above, R¹ represents a hydrogen atom or monovalent group, and two R¹s may be the same or different.]

R¹ in formula (12) represents a monovalent group. The monovalent group is preferably an alkyl group, an alkoxy group, an aryl group, a halogen atom, a heterocyclic group, or a group having an ester structure. The definition and specific examples of the alkyl group, the alkoxy group, the aryl group, the halogen atom, the heterocyclic group, and the group having an ester structure are the same as the definition and specific examples in the R described above.

The fullerene derivative of the present invention has one or more structures selected from the group consisting of the structure represented by formula (2-1) and the structure represented by formula (2-2). The fullerene derivative preferably has one to three structures described above and more preferably has one or two structures described above.

In formula (2-1) and formula (2-2), ring D represents an aromatic ring. Specific examples of the aromatic ring include a benzene ring, a naphthalene ring, an anthracene ring, a phenanthrene ring, and a pyrene ring. Among those, a benzene ring is preferable.

The structure represented by formula (2-1) is preferably a structure represented by formula (5).

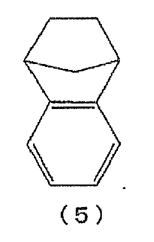

Specific examples of the fullerene derivative of the present invention include the following compounds.

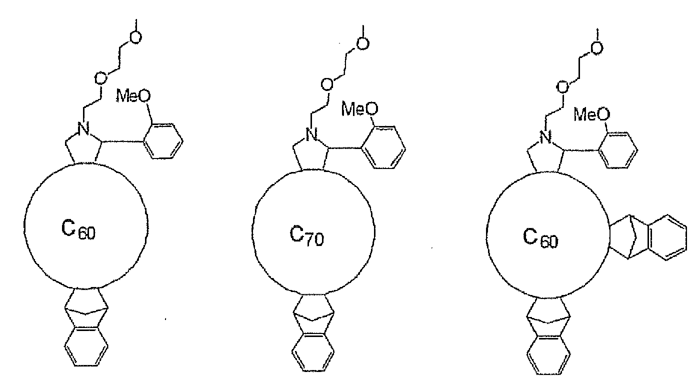

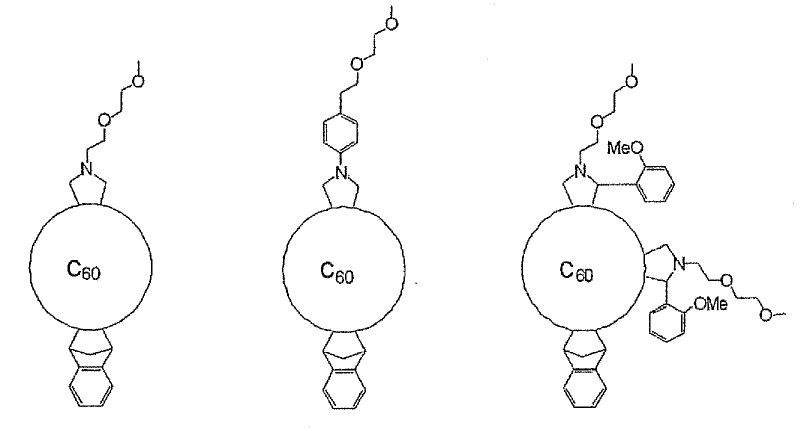

In the above compounds, C₆₀ ring and C₇₀ ring each represent a fullerene ring having 60 and 70 carbon atoms.

Among the fullerene derivatives of the present invention, the fullerene derivative represented by formula (3-1), the fullerene derivative represented by formula (3-2), the fullerene derivative represented by formula (3-3), and the fullerene derivative represented by formula (3-4) are preferable. 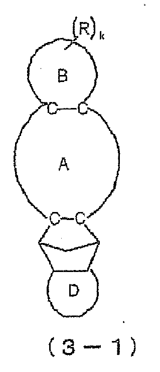 [wherein, ring A, ring B, ring D, R, and k represent as defined above.] 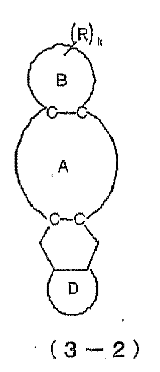 [wherein, ring A, ring B, ring D, R, and k represent as defined above.] 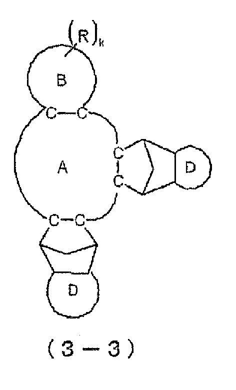 [wherein, ring A, ring B, ring D, R, and k represent as defined above, and a plurality of rings D may be the same or different.] 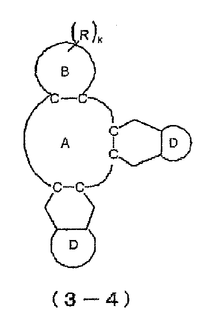 [wherein, ring A, ring B, ring D, R and k represent as defined above, and a plurality of rings D may be the same or different.

The fullerene derivatives of the present invention can be produced, for example, by the methods shown below. 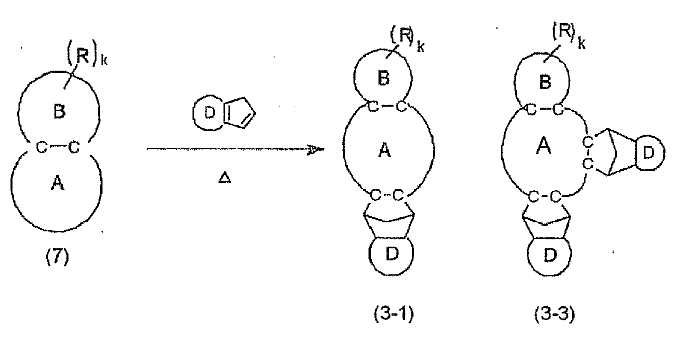 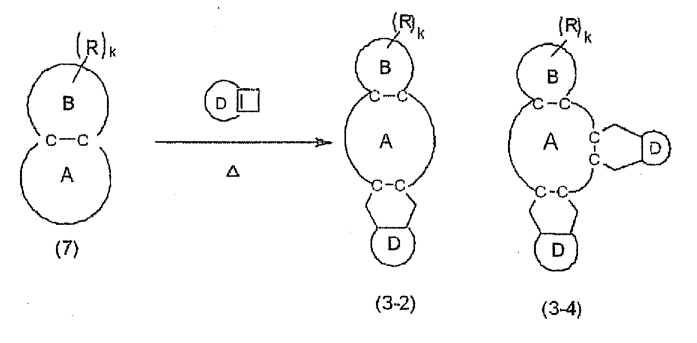 (wherein, ring A, ring B, ring D, R, and k represent as defined above.)

In other words, an aromatic compound such as indene is added to the compound represented by formula (7) by thermal cyclization, to produce the fullerene derivative represented by formula (3-1) and the fullerene derivative represented by formula (3-3). The separation of the fullerene derivative represented by formula (3-1) and the fullerene derivative represented by formula (3-3) can be carried out using column chromatography.

In addition, an aromatic compound such as benzocyclobutene is added to the compound represented by formula (7) by thermal cyclization, to produce the fullerene derivative represented by formula (3-2) and the fullerene derivative represented by formula (3-4). The separation of the fullerene derivative represented by formula (3-2) and the fullerene derivative represented by formula (3-4) can be carried out using column chromatography.

The compound represented by formula (7) used for the above methods includes a fullerene derivative in which ring B that can be synthesized by aza 1,3-dipolar addition reaction is a pyrrolidine ring, a fullerene derivative in which ring B is a pyrazoline ring, a fullerene derivative in which ring B is an isoxazoline ring, and the like.

The thermal cycloaddition reaction in the above methods is carried out in the thermal conditions from generally 100°C to 180°C or so. The reaction solvent has a boiling point of the reaction temperature or so, favorably dissolves the raw material compound (7), is preferably a solvent stable to heating, and includes aromatic hydrocarbons such as toluene, xylene, mesitylene, monochlorobenzene, and o-dichlorobenzene, and the like.

Other methods of producing the fullerene derivative of the present invention include the following methods. 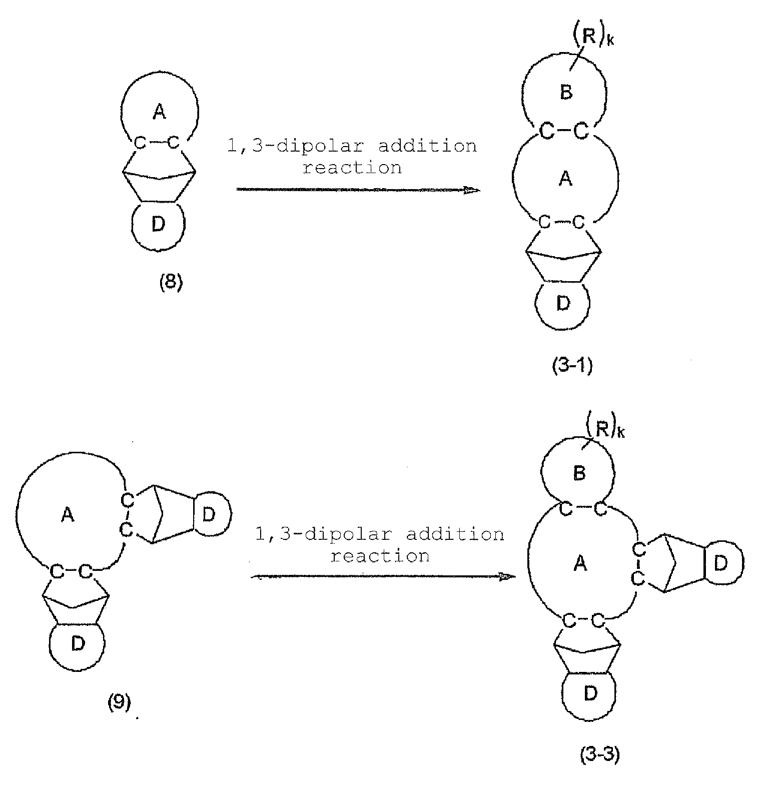 (wherein, ring A, ring B, ring D, R, and k represent as defined above.)

In other words, an aromatic compound such as indene is added to the fullerene such as C60 and C70 by thermal cyclization, and a compound having k number of the substituent R is added to the obtained adduct (8) or adduct (9) by addition reaction such as 1,3-dipolar addition reaction to form a ring B, to produce the fullerene derivative represented by formula (3-1) or the fullerene derivative represented by formula (3-3). When ring B is a ring having 3 carbon atoms, the adduct (8) or adduct (9) are reacted with a diazo compound, to produce the fullerene derivative represented by formula (3-1) or the fullerene derivative represented by formula (3-3).

In the above method, the compound represented by formula (8) can be synthesized by adding an aromatic compound such as indene to the fullerene such as C60 and C70 by thermal cycloaddition. At that time, the compound represented by formula (9) is produced, in addition to the compound represented by formula (8), and these compounds can be separated by column chromatography.

In addition, an aromatic compound such as benzocyclobutene is added to the fullerene such as C60 and C70 by thermal cyclization, and the same reaction as the above method is carried out using the obtained adduct (8') or adduct (9'), and thus the fullerene derivative represented by formula (3-2) or the fullerene derivative represented by formula (3-4) can be produced. 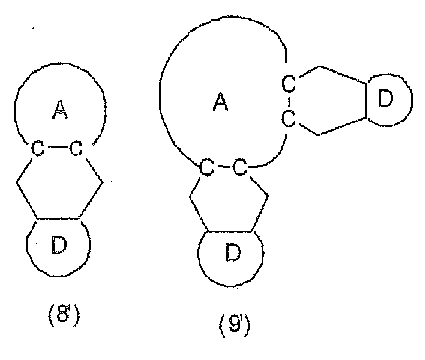 (wherein, ring A, ring B, and ring D represent as defined above.)

Since the fullerene derivative can be used for an organic photoelectric conversion element together with an electron-donating compound, the present invention also provides a composition comprising the fullerene derivative and an electron-donating compound.

The electron-donating compound is preferably, polymer compounds, for example, polyvinylcarbazole and its derivative, polysilane and its derivative, a polysiloxane derivative having an aromatic amine in its side chain or main chain, polyaniline and its derivative, polythiophene and its derivative, polypyrrole and its derivative, polyphenylenevinylene and its derivative, polythienylenevinylene and its derivative, and polyfluorene and its derivative, from the viewpoint that the composition is coated and used.

In addition, the electron-donating compound is preferably the polymer compound having a repeating unit selected from the group consisting of formula (10) and formula (11) and more preferably the polymer compound having a repeating unit represented by formula (10), from the viewpoint of conversion efficiency. 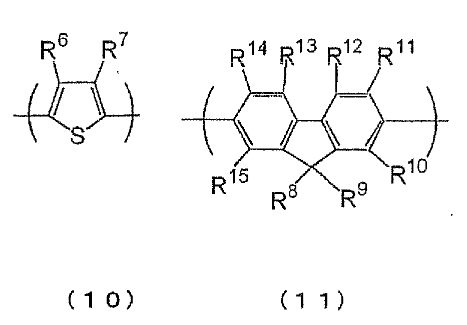 [wherein, R⁶ R⁷, R⁸ R⁹, R¹⁰, R¹¹, R¹², R¹³, R¹⁴, and R¹⁵ are the same or different, and represent a hydrogen atom, an alkyl group, an alkoxy group, or an aryl group.]

In formula (10), specific examples when R⁶ and R⁷ are an alkyl group include the alkyl groups described above for R. Specific examples when R⁶ and R⁷ are an alkoxy group include the alkoxy groups described above for R. Specific examples when R⁶ and R⁷ are an aryl group include the aryl groups described above for R.

In formula (10), at least one of R⁶ and R⁷ is preferably an alkyl group having 1 to 20 carbon atoms and more preferably an alkyl group having 4 to 8 carbon atoms, from the viewpoint of conversion efficiency.

In formula (11), specific examples when R⁸ to R¹⁵ are an alkyl group include the alkyl groups described above for R. Specific examples when R⁸ to R¹⁵ are an alkoxy group include the alkoxy groups described above for R. Specific examples when R⁸ to R¹⁵ are an aryl group include the aryl groups described above for R.

In formula (11), R¹⁰ to R¹⁵ are preferably a hydrogen atom, from the viewpoint of ease of performing synthesis of a monomer. In addition, R⁸ and R⁹ are preferably an alkyl group having 1 to 20 carbon atoms or an aryl group having 6 to 20 carbon atoms and more preferably an alkyl group having 5 to 8 carbon atoms or an aryl group having 6 to 15 carbon atoms, from the viewpoint of conversion efficiency.

The ful lerene derivative contained in the composition of the present invention is preferably 10 to 1000 parts by weight and more preferably 50 to 500 parts by weight, based on 100 parts by weight of the electron-donating compound.

The present invention also provides an organic photoelectric conversion element having a pair of electrodes, at least one of the electrodes being transparent or translucent, and a layer between the electrodes, that contains the fullerene derivative used in the present invention. The fullerene derivative used in the present invention can be used either as an electron-accepting compound or as an electron-donating compound, and preferably used as an electron-accepting compound.

Next, the operation mechanism of the organic photoelectric conversion element is described. Photoenergy incident from the transparent or translucent electrode is absorbed by the electron-accepting compound and/or the electron-donating compound to generate excitons each composed of an electron and a hole bound to each other. When the generated excitons move and reach the heterojunction interface where the electron-accepting compound and the electron-donating compound are adjacent to each other, electrons and holes separate, due to the difference between the HOMO energy and the LUMO energy in each of these compounds at the interface, to generate charges (electrons and holes) that can move independently. The generated charges each move to the electrodes, and hence the charges can be taken out as electrical energy (current) to the outside.

As the organic photoelectric conversion element of the present invention, either of 1. an organic photoelectric conversion element comprising a pair of electrodes, at least one of the electrodes being transparent or translucent, a first layer disposed between the electrodes and containing the fullerene derivative of the present invention as an electron-accepting compound, and a second layer disposed adjacent to the first layer and containing an electron-donating compound; and 2. an organic photoelectric conversion element having a pair of electrodes, at least one of the electrodes being transparent or translucent, and one or more layer disposed between the electrodes and containing the fullerene derivative of the present invention as an electron-accepting compound and an electron-donating compound is preferable.

From the viewpoint of including many heterojunction interfaces, the organic photoelectric conversion element of the above item 2 is preferable. Also, in the organic photoelectric conversion element of the present invention, an additional layer may be disposed between the electrode and the layer containing the fullerene derivative used in the present invention. Examples of the additional layer include a charge transport layer which transports holes or electrons.

In the organic photoelectric conversion element of the above item 2, the amount of the fullerene derivative in the organic layer containing the fullerene derivative and the electron-donating compound is preferably 10 to 1000 parts by weight and more preferably 50 to 500 parts by weight, based on 100 parts by weight of the electron-donating compound.

The layer containing the fullerene derivative used in the organic photoelectric conversion element of the present invention is preferably formed of an organic thin film containing the fullerene derivative. The thickness of the organic thin film is generally 1 nm to 100 µm, preferably 2 nm to 1000 nm, more preferably 5 nm to 500 nm, and further preferably 20 µm to 200 nm.

The organic photoelectric conversion element of the present invention is generally formed on a substrate. This substrate has only to be a substrate that does not undergo any chemical change when electrodes are formed and a layer of an organic substance is formed. Examples of the material of the substrate include glass, plastic, polymer film, and silicon. In the case of an opaque substrate, the opposite electrode (in other words, the electrode far from the substrate) is transparent or translucent.

The material of the transparent or translucent electrode includes a conductive metal oxide film, a translucent metal thin film, and the like. Specifically, the films prepared using conductive materials comprising indium oxide, zinc oxide, tin oxide, and indium tin oxide (ITO), which are composite materials thereof, and indium zinc oxide and the like (such as NESA), gold, platinum, silver, copper, and the like are used, and ITO, indium zinc oxide, and tin oxide are preferable. The method for preparing the electrode includes a vacuum deposition method, a sputtering method, an ion plating method, a plating method, and the like.

In addition, as the electrode materials, organic transparent conductive films of polyaniline and derivatives thereof, and polythiophene and derivatives thereof may also be used.

As the electrode materials, one electrode of the pair of electrodes is preferably a material having a small work function. The electrode containing a material having a small work function may be transparent or translucent. Examples of the material used are metals such as lithium, sodium, potassium, rubidium, cesium, magnesium, calcium, strontium, barium, aluminum, scandium, vanadium, zinc, yttrium, indium, cerium, samarium, europium, terbium, and ytterbium; alloys of two or more of these metals; alloys of one or more of these metals and one or more of gold, silver, platinum, copper, manganese, titanium, cobalt, nickel, tungsten, and tin; graphite; or graphite intercalation compounds. Examples of the alloys include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.

The additional layer that may be disposed between the electrode and the layer containing the fullerene derivative used in the present invention may be a buffer layer, and the material used as the buffer layer includes alkali metals such as lithium fluoride, halides of alkali earth metals, oxides such as titanium oxide, and the like. In addition, in case of using an inorganic semiconductor, it can be also used in the form of fine particles.

The method for producing the organic thin film is not particularly limited, and examples include a method for forming a film from a solution containing the fullerene derivative used in the present invention.

The solvent used for the film formation from a solution is not particularly limited as long as the solvent dissolves the fullerene derivative used in the present invention. Examples of this solvent include: hydrocarbon solvents such as toluene, xylene, mesitylene, tetralin, decalin, bicyclohexyl, butylbenzene, sec-butylbenzene, and tert-butylbenzene; halogenated saturated hydrocarbon solvents such as carbon tetrachloride, chloroform, dichloromethane, dichloroethane, chlorobutane, bromobutane, chloropentane, bromopentane, chlorohexane, bromohexane, chlorocyclohexane, and bromocyclohexane; halogenated unsaturated hydrocarbon solvents such as chlorobenzene, dichlorobenzene, and trichlorobenzene; and ether solvents such as tetrahydrofuran and tetrahydropyran. Generally, the fullerene derivative can be dissolved in the above solvents in an amount of 0.1% by weight or more.

The solution may further contain a polymer compound. Specific examples of the solvent used for the solution include the above-described solvents; and, from the viewpoint of the solubility of the polymer compound, aromatic hydrocarbon solvents are preferable, and toluene, xylene, and mesitylene are more preferable.

For the film formation from a solution, the coating methods such as spin coating, casting, microgravure coating, gravure coating, bar coating, roll coating, wire bar coating, dip coating, spray coating, screen printing, flexographic printing, offset printing, ink jet printing, dispenser printing, nozzle coating, and capillary coating can be used; and spin coating, flexographic printing, ink jet printing, and dispenser printing are preferable.

The organic photoelectric conversion element generates photovoltaic power between the electrodes by the irradiation from the transparent or translucent electrode with light such as sunlight, and thus may be operated as an organic thin film solar cell. It can be also used as an organic thin film solar cell module by integrating a plurality of organic thin film solar cells.

Also, photocurrent is allowed to flow by the irradiation from the transparent or translucent electrode with light under the condition that a voltage is applied between the electrodes, and thus may be operated as an organic optical sensor. It can be also used as an organic image sensor by integrating a plurality of organic optical sensors.

### Examples

Hereinafter, examples are presented for describing the present invention in further detail, but the present invention is not limited to these examples.

MALDI-TOF MS spectra were measured using Reflex III manufactured by Bruker Corp.

### Synthesis Example 1 (Synthesis of Fullerene Derivative A and Fullerene Derivative B)

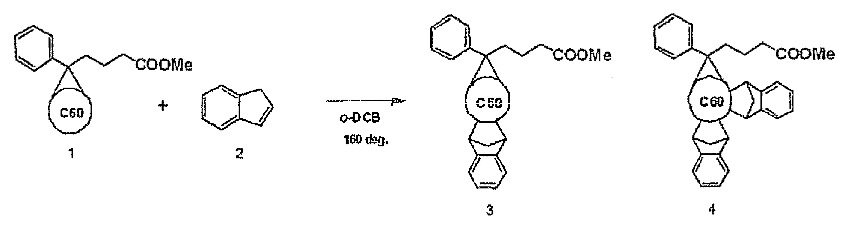

Under a nitrogen atmosphere, 0. 68 g (0.75 mmol) of C60PCBM (compound 1) and 50 ml of o-dichlorobenzene were charged into a 50 ml-recovery flask, and 1. 04 g (9.0 mmol) of indene (compound 2) was added thereto, then the mixture was heated and stirred at an internal temperature of 160°C for 24 hours under a nitrogen stream. The reaction solution was cooled and thereafter concentrated, the residue was purified in the conditions of silica gel column chromatography (using 200 g of WakosilC-300, developing solution: hexane/toluene=7:1 → 1:1 (v/v)), and the resulting two fractions were each concentrated to 10 ml under reduced pressure. Thereafter, 20 ml of methanol was added to the concentrated solution, and the generated precipitate was filtered and washed twice using 10 ml of methanol, and then dried under reduced pressure at 6700 Pa and 80C° for 5 hours, to obtain fullerene derivative A (compound 3) and fullerene derivative B (compound 4) as an isomer mixture.

From the first fraction containing the fullerene derivative A as a main component, 0.07 g of a product was obtained. As a result of analyzing the product by HPLC, the composition of the fullerene derivative obtained from the surface percentage of the chromatogram was 97.1% of the fullerene derivative A and 2.2% of the fullerene derivative B.

From the second fraction containing the fullerene derivative B as a main component, 0. 29 g of a product was obtained. As a result of analyzing the product by HPLC, the composition of the fullerene derivative obtained from the surface percentage of the chromatogram was 43.7% of the fullerene derivative A and 56.1% of the fullerene derivative B.

Analysis by HPLC uses L-Column ODS (manufactured by Chemicals Evaluation and Research Institute, Japan, 4.6 mm × 25 cm, film thickness: 3 µm) as a column, the column was set at a detection wavelength of 355 nm and a flow amount of 1 ml/min. Using methanol containing 50 mM ammonium acetate (solution A) and toluene (solution B) as a solvent, the analysis was performed with a gradient of 20% to 60% of solution B over 40 minutes.

The first fraction: ¹H-NMR (270MHz/CDCl3): δ 1..80-3.00 (m), 3.50-3.75 (m), 4.60-5.20 (m), 7.10-8.30 (m) Synthesis Example 2 (Synthesis of Fullerene Derivative C and Fullerene Derivative D)

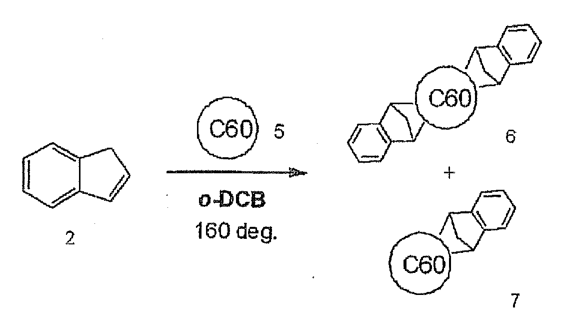

Under a nitrogen atmosphere, 0.58 g (5.00 mmol) of indene (compound 2) and 50 ml of o-dichlorobenzene were charged into a 50 ml-recovery flask, and 0.30 g (0.42 mmol) of C60 fullerene (compound 5) was added thereto, then the mixture was heated and stirred at an internal temperature of 160°C for 20 hours under a nitrogen stream. The reaction solution was cooled and thereafter concentrated with an evaporator, the residue was purified by silica gel column chromatography (using 300 g of WakosilC-300, developing solution: hexane/toluene=7:1 → 1:1 (v/v)), and the resulting fractions were each concentrated to 10 ml under reduced pressure. Thereafter, 30 ml of methanol was added thereto, and the generated precipitate was filtered and washed twice with 10 ml of methanol, and then dried under reduced pressure at 6700 Pa and 80°C for 5 hours, to obtain 0.05 g of fullerene derivative C (compound 6) and 0.10 g of fullerene derivative D (compound 7).

### Comparative Example 1 (Preparation and Evaluation of Organic Thin Film Solar Cells)

As an electron donor, regioregular poly(3-hexylthiophene) (manufactured by Aldrich Corp., Lot No. : 09007KH) was dissolved in chlorobenzene in a concentration of 1% (% by weight). Thereafter, the fullerene derivative A was mixed as an electron acceptor into the solution in a weight equivalent to the weight of the electron donor. Then, 1 part by weight of silica gel (manufactured by Wako Pure Chemical Industries, Wakogel C-300, particle diameter: 45 to 75 µm) was added to 100 parts by weight of the solution as an adsorbent, and the mixture was stirred for 12 hours. Subsequently, the resulting mixture was filtered with a Teflon (trade mark) filter of 1.0 µm in pore size, to prepare a coating solution.

A glass substrate on which an ITO film with a thickness of 150 nm was provided by sputtering was subjected to ozone-UV treatment to perform surface treatment. Next, the coating solution was coated by spin-coating, to obtain an active layer (film thickness: about 100 nm) of an organic thin film solar cell. Then, the coated substrate was baked under the conditions of 90°C under vacuum for 60 minutes. Then, lithium fluoride was vapor-deposited in a thickness of 4 nm with a vacuum deposition apparatus, and subsequently, Al was vapor-deposited in a thickness of 100 nm. The degree of vacuum upon vapor deposition was all 1 to 9 × 10⁻³ Pa. Also, the shape of the resulting organic thin film solar cell was 2 mm × 2 mm regular tetragon. The open circuit voltage (Voc) of the resulting organic thin film solar cells was obtained by measuring the current and voltage generated by irradiating a certain amount of light by using a solar simulator (manufactured by Bunkoukeiki Co., Ltd., trade name: "OTENTO-SUN II": AM 1.5 G filter, irradiance: 100 mW/cm²). The results are shown in Table 1. Comparative Example 2 (Preparation and Evaluation of Organic Thin Film Solar Cells)

The same procedure was carried out as in Comparative Example 1 except using the fullerene derivative B in place of the fullerene derivative A in Comparative Example 1, to measure. The results are shown in Table 1.

### Example 1 (Synthesis of Fullerene Derivative E and Fullerene Derivative F)

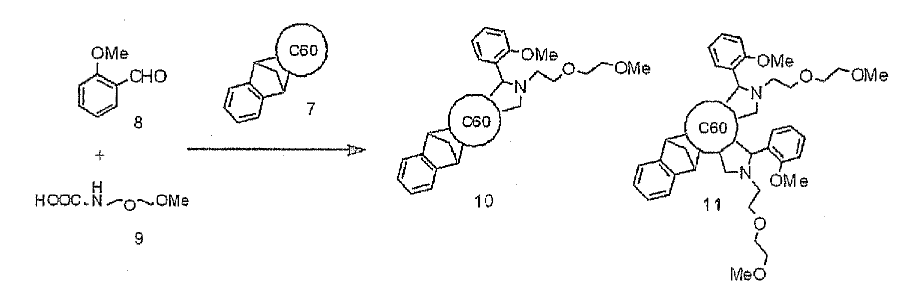

Under a nitrogen atmosphere, 0.07 g (0.51 mmol) of 2-methoxybenzaldehyde (compound 8), 0.07 g (0.40 mmol) of [2- (2-methoxyethoxy)ethylamino] acetic acid (compound 9), 0.20 g (0.23 mmol) of the fullerene derivative D, and 30 ml of chlorobenzene were charged into a 50 ml-recovery flask, and the mixture was heated and stirred at a temperature of 130°C for 8 hours under a nitrogen stream. The reaction solution was cooled to room temperature and then concentrated under reduced pressure with an evaporator, and thereafter the resulting residue was purified by silica gel column chromatography (WakosilC-300, developing solution: toluene/ethyl acetate=100:0 → 90:10 (volume ratio)), and concentrated to a total volume of 10 ml with an evaporator, and 20 ml of methanol was added thereto, to obtain a precipitate. This precipitate was washed with 10 ml of methanol and dried under reduced pressure, to obtain 66 mg of compound 10 (fullerene derivative E), target object, as an isomer mixture (yield: 25.4%).

The fullerene derivative E (compound 10) was fractionated and then further developed changing the developing solution of silica gel column chromatography to a mixed solvent mixing toluene and ethyl acetate at a volume ratio of 1:1, and a fraction was concentrated. Then, the residue was washed with 10 ml of methanol and dried under reduced pressure, to obtain 66 mg of a fullerene derivative containing two or more N-methoxyethoxyethylpyrrolidine structures, as an isomer mixture. The isomer mixture is called fullerene derivative mixture 1. The fullerene derivative mixture 1 contains fullerene derivative F (compound 11).

The result of analyzing the fullerene derivative E and the fullerene derivative F with MALDI-TOF MS was shown below.

Fullerene Derivative E: MALDI-TOF-MS (matrix: SA) found 1087.2 (calcd for C₈₃H₂₉NO₃: 1087.2)

Fullerene Derivative Mixture 1: MALDI-TOF-MS (matrix: SA) found
1338.4 (calcd for C₉₇H₅₀N₂O₆: 1338.4)

It was confirmed that the fullerene derivative mixture 1 contains the fullerene derivative F.

### Example 2 (Preparation and Evaluation of Organic Thin Film Solar Cells)

The same procedure was carried out as in Comparative Example 1 except using the fullerene derivative E in place of the fullerene derivative A in Comparative Example 1, to measure. The results are shown in Table 1.

### Example 3 (Synthesis of Fullerene Derivative G and Fullerene Derivative H)

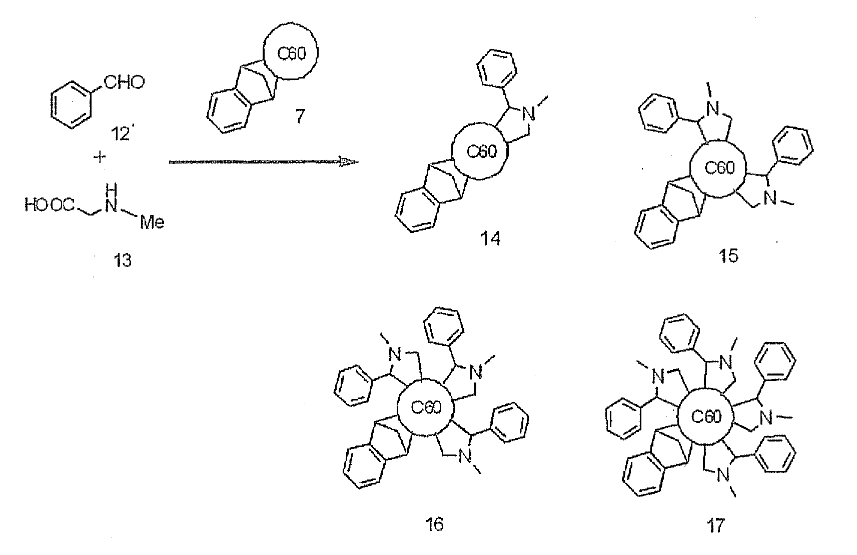

Under a nitrogen atmosphere, 0.04 g (0.38 mmol) of benzaldehyde (compound 12), 0.03 g (0.34 mmol) of N-methylglycine (compound 13), 0.18 g (0.22 mmol) of the fullerene derivative D (compound 7), and 30 ml of chlorobenzene were charged into a 50 ml-recovery flask, and the mixture was heated and stirred at a temperature of 130°C for 8 hours under a nitrogen stream. The reaction solution was cooled to room temperature (25°C) and then concentrated under reduced pressure with an evaporator, and thereafter the resulting residue was purified by silica gel column chromatography (WakosilC-300, developing solution: toluene/ethyl acetate=100:0 → 90:10 (volume ratio)), and concentrated to a total volume of 10 ml with an evaporator, and 20 ml of methanol was added thereto, to obtain a precipitate. This precipitate was washed with 10 ml of methanol and dried under reduced pressure, to obtain 42 mg of a fullerene derivative mixture 2. The fullerene derivative mixture 2 contains an isomer mixture of compound 14 and an isomer mixture of compound 15. The fullerene derivative mixture 2 was fractionated and then further developed changing the developing solution of silica gel column chromatography to a mixed solvent mixing toluene and ethyl acetate at a volume ratio of 1:1, and a fraction was concentrated. Then, the residue was washed with 10 ml of methanol and dried under reduced pressure, to obtain 145 mg of a fullerene derivative mixture 3. The fullerene derivative mixture 3 contains an isomer mixture of compound 14, an isomer mixture of compound 15, an isomer mixture of compound 16, and an isomer mixture of compound 17.

For the resulting mixtures, MALDI-TOF-MS spectra were each measured using Voyager-DE STR manufactured by Applied Biosystems.
(Fullerene Derivative Mixture 2): MALDI-TOF (matrix: sinapic acid) found for [M+H]⁺: 970.2 (n=1, calcd. for M⁺: 969.15), 1103.3 (n=2, calcd. for M⁺: 1102.24)
(Fullerene Derivative Mixture 3): MALDI-TOF (matrix: sinapic acid) found for [M+H]⁺: 970.3 (n=1, calcd. 969.15), 1103.4 (n=2, calcd. for M⁺: 1102.24), 1236.6 (n=3, calcd. for M⁺: 1235.33), 1369.7 (n=4, calcd. for M⁺: 1368.42)

### Comparative Example 3 (Preparation and Evaluation of Organic Thin Film Solar Cells)

The same procedure was carried out as in Comparative Example 1 except using [60]-PCBM (Phenyl C61-butyric acid methyl ester, manufactured by Frontier Carbon Corporation, trade name: E100, Lot No.: 8A0125A) in place of the fullerene derivative A in Comparative Example 1, to measure. The results are shown in Table 1.

### Comparative Example 4 (Preparation and Evaluation of Organic Thin Film Solar Cells)

The same procedure was carried out as in Comparative Example 1 except using the fullerene derivative C in place of the fullerene derivative A in Comparative Example 1, to measure. The results are shown in Table 1.

### Comparative Example 5 (Preparation and Evaluation of Organic Thin Film Solar Cells)

The same procedure was carried out as in Comparative Example 1 except using the fullerene derivative D in place of the fullerene derivative A in Comparative Example 1, to measure. The results are shown in Table 1.

### Example 4 (Preparation and Evaluation of Organic Thin Film Solar Cells)

The same procedure was carried out as in Comparative Example 1 except using the fullerene derivative mixture 3 in place of the fullerene derivative A in Comparative Example 1, to measure. The results are shown in Table 1.

**Table 1**

| | Fullerene Derivative | Open Circuit Voltage (Voc) |
|---|---|---|
| Example 2 | Fullerene Derivative E | 0.86 |
| Example 4 | Fullerene Derivative Mixture 3 | 0.91 |
| Comparative Example 1 | Fullerene Derivative A 0.81 | |
| Comparative Example 2 | Fullerene Derivative B | 0.83 |
| Comparative Example 3 | [60] - PCBM | 0.51 |
| Comparative Example 4 | Fullerene Derivative C | 0.72 |
| Comparative Example 5 | Fullerene Derivative D | 0.55 |

### Industrial Applicability

Since the fullerene derivative of the present invention is applicable to an organic photoelectric conversion element having a high open circuit voltage (Voc), it is suitable for an organic thin film solar cell or organic image sensor and is very useful.

## Claims

1. A fullerene derivative comprising a structure represented by formula (1) and one or more structures selected from the group consisting of a structure represented by formula (2-1) and a structure represented by formula (2-2). 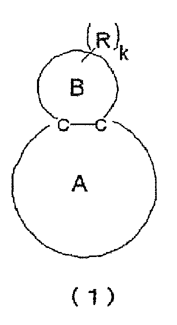 [wherein, ring A represents a fullerene skeleton, ring B represents a heterocyclic ring having a carbon number of 3 to 6, R represents a monovalent group, and k represents an integer of 1 to 8; and when there is a plurality of Rs, the Rs may be the same or different.] 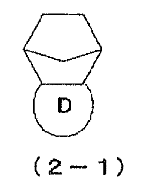 [wherein, ring D represents an aromatic ring.] 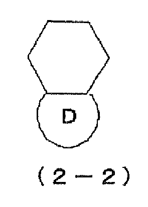 [wherein, ring D represents an aromatic ring.]

2. The fullerene derivative according to Claim 1 represented by formula (3-1). 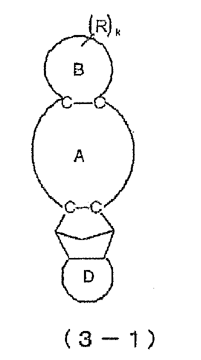 [wherein, ring A, ring B, ring D, R, and k represent as defined above.]

3. The fullerene derivative according to Claim 1 represented by formula (3-2). 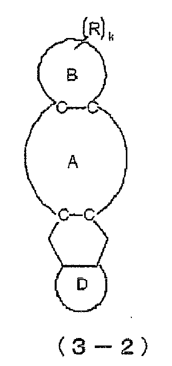 [wherein, ring A, ring B, ring D, R, and k represent as defined above.]

4. The fullerene derivative according to Claim 1 represented by formula (3-3). 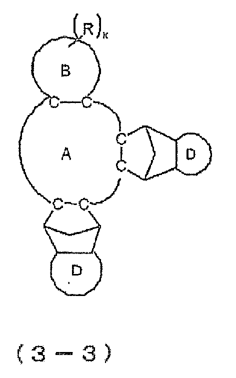 [wherein, ring A, ring B, ring D, R, and k represent as defined above, and a plurality of rings D may be the same or different.]

5. The fullerene derivative according to Claim 1 represented by formula (3-4). 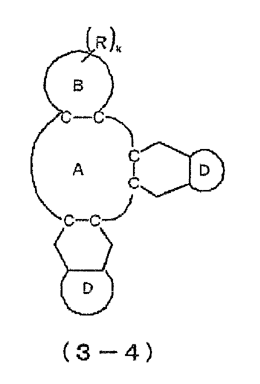 [wherein, ring A, ring B, ring D, R, and k represent as defined above, and a plurality of rings D may be the same or different.]

6. The fullerene derivative according to any of Claims 1 to 5, wherein the structure represented by formula (1) is a structure represented by formula (12). 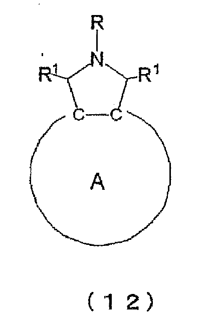 [wherein, ring A and R represent as defined above, R¹ represents a hydrogen atom or monovalent group, and two R¹s may be the same or different.]

7. The fullerene derivative according to any of Claims 1 to 6, wherein ring D is a benzene ring.

8. The fullerene derivative according to any of Claims 1 to 7, wherein the monovalent group represented by R is an alkyl group, an alkoxy group, an aryl group, a halogen atom, a heterocyclic group, or a group having an ester structure.

9. The fullerene derivative according to Claim 8,
wherein the monovalent group represented by R is an alkyl group having 1 to 20 carbon atoms, an alkoxy group having 1 to 20 carbon atoms, an aryl group having 6 to 60 carbon atoms, a halogen atom, or a heterocyclic group that is a thienyl group, a pyridyl group, a furyl group, a quinolyl group, an isoquinolyl group, a pyrrolyl group, or a piperidyl group, or a group having an ester structure represented by formula (6). 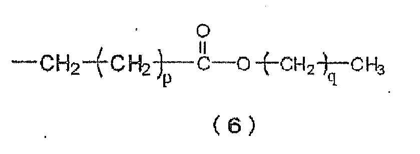 [wherein, p represents an integer of 0 to 10, and q is an integer of 0 to 10.].

10. The fullerene derivative according to Claim 1 that is any of the following compounds. 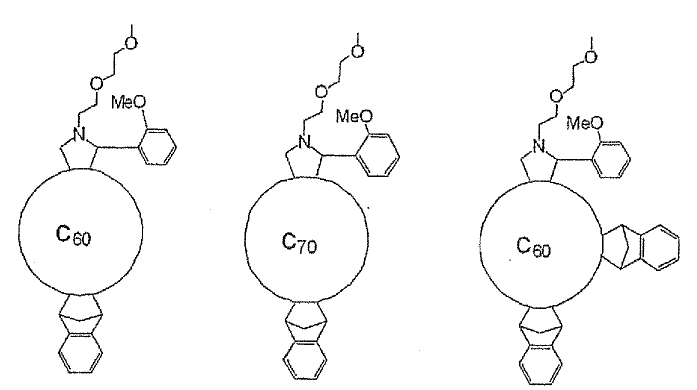 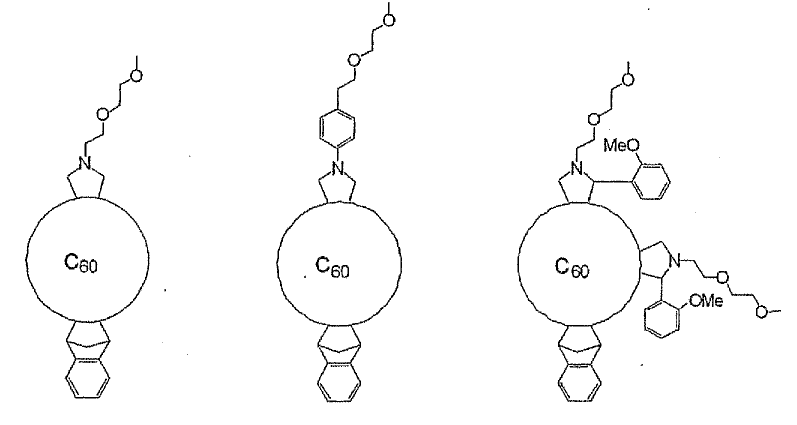 [wherein, the C₆₀ ring and C₇₀ ring each represent a fullerene ring having 60 and 70 carbon atoms.]

11. A composition comprising the fullerene derivative as defined in Claim 1 and an electron-donating compound.

12. The composition according to Claim 10, wherein the electron-donating compound is a polymer compound.

13. A compound comprising the fullerene derivative as defined in Claim 10 and an electron-donating compound, the electron-donating compound being a polymer compound having a repeating unit selected from the group consisting of formula (10) and formula (11). 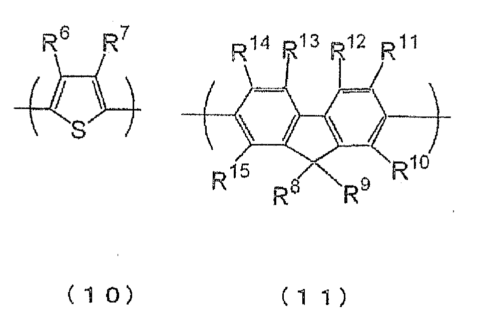 [wherein, R⁶, R⁷, R⁸, R⁹, R¹⁰, R¹¹, R¹², R¹³, R¹⁴, and R¹⁵ are the same or different, and represent a hydrogen atom, an alkyl group having 1 to 20 carbon atoms, an alkoxy group having 1 to 20 carbon atoms, or an aryl group having 6 to 60 carbon atoms.]

14. An organic photoelectric conversion element comprising a pair of electrodes, at least one of the electrodes being transparent or translucent, and a layer between the electrodes, that contains the fullerene derivative as defined in Claim 1.

15. An organic photoelectric conversion element comprising a pair of electrodes, at least one of the electrodes being transparent or translucent, and a layer between the electrodes, that contains the fullerene derivative as defined in Claim 10.

16. An organic photoelectric conversion element comprising a pair of electrodes, at least one of the electrodes being transparent or translucent, and a layer between the electrodes, that contains the compound as defined in Claim 11.

17. An organic photoelectric conversion element comprising a pair of electrodes, at least one of the electrodes being transparent or translucent, and a layer between the electrodes, that contains the compound as defined in Claim 13.
